(19) 

Europäisches
Patentamt
European
Patent Office
Office européen
des brevets

(11) **EP 3 726 273 A1**

(12) **EUROPEAN PATENT APPLICATION**
published in accordance with Art. 153(4) EPC

(43) Date of publication:
**21.10.2020 Bulletin 2020/43**

(21) Application number: **18889812.6**

(22) Date of filing: **13.11.2018**

(51) Int Cl.:
**G02B 21/00** (2006.01)          **C12M 1/34** (2006.01)
**G02B 21/06** (2006.01)          **G02B 21/26** (2006.01)

(86) International application number:
**PCT/JP2018/041976**

(87) International publication number:
**WO 2019/116807 (20.06.2019 Gazette 2019/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.12.2017 JP 2017240539**

(71) Applicant: **Fujifilm Corporation
Minato-ku
Tokyo 106-8620 (JP)**

(72) Inventor: **MATSUBARA, Kenta
Ashigarakami-gun, Kanagawa 258-8538 (JP)**

(74) Representative: **Klunker IP
Patentanwälte PartG mbB
Destouchesstraße 68
80796 München (DE)**

(54) **OBSERVATION DEVICE, OBSERVATION METHOD, AND OBSERVATION PROGRAM**

(57)       An observation apparatus, an observation method, and an observation program capable of capturing an image close to an appropriate imaging position even in a case where a scanning trajectory is shifted are provided. The observation apparatus includes an imaging unit 37 that images an observation target accommodated in a cultivation container 20, a movement unit 12 that scans the observation target by relatively moving the cultivation container 20 with respect to the imaging unit 37 in accordance with a scheduled trajectory, a measurement unit 38 that acquires measured shape information of the cultivation container 20 by measuring the cultivation container 20 along a scanning trajectory using an observation region, a storage unit 45 that stores reference shape information obtained by measuring a shape of the cultivation container 20 using the measurement unit 38 along a reference trajectory which is a reference in a case where the shape of the cultivation container 20 is measured, a calculation unit 46 that calculates a shift of a scanning trajectory M with respect to a reference trajectory K based on the reference shape information and the measured shape information, and a control unit 40 that corrects the scheduled trajectory based on the shift and scans the imaging unit 37 based on the corrected scheduled trajectory.

FIG. 1

EP 3 726 273 A1

**Description**

**BACKGROUND OF THE INVENTION**

1. Field of the Invention

The technology of the disclosure relates to an observation apparatus, an observation method, and an observation program for observing an observation target by relatively moving at least one of a container accommodating the observation target or an imaging unit with respect to the other.

2. Description of the Related Art

In recent years, various technologies for imaging an observation target such as various cells and analyzing an acquired image have been suggested. For example, a method of imaging a pluripotent stem cell such as an embryonic stem (ES) cell and an induced pluripotent stem (iPS) cell, a differentiation-induced cell, or the like using a microscope and determining a differentiation state or the like of the cell by recognizing a feature of the image has been suggested. For example, JP2016-149998A discloses an identification apparatus that can suitably specify an observation target even in a case where an imaging condition or the like changes. In addition, JP2016-099592A discloses a microscope that adjusts refraction of illumination light caused by a liquid surface shape of a solution for culturing a cell. In addition, WO2008/146474A discloses an observation apparatus that can recognize an amount of a culture medium for culturing a cell.

The pluripotent stem cell such as the ES cell and the iPS cell has a capability to differentiate into cells of various tissues and has drawn attention for its applicability in regenerative medicine, development of medication, identification of diseases, and the like.

In a case where the cell is imaged as described above, it is known that the cell as the observation target is scanned and measured by the microscope and is determined from an obtained image. In order to implement industrialization of regenerative medicine or multilevel experiment in drug discovery research, it is important to perform imaging at a high speed and determine quality at a high speed.

In a case where a cell that is cultured using a cultivation container such as a well plate, a Petri dish, and a flask is observed, coordinates at which scanning and measurement are performed are decided in accordance with a shape and a dimension of the cultivation container. The microscope is moved along a scanning trajectory that passes through the decided coordinates.

**SUMMARY OF THE INVENTION**

In a case where imaging is performed along the decided scanning trajectory, an unintended image may be captured. For example, in a case where the cultivation container is not correctly placed on the placing stand, the scanning trajectory with respect to the cultivation container is shifted in imaging using the coordinates which are decided by regarding the cultivation container as being at a correct position, and the accommodated cell cannot be appropriately imaged.

The technology of the disclosure is conceived in view of the above point, and an object thereof is to provide an observation apparatus, an observation method, and an observation program capable of capturing an image close to an appropriate imaging position even in a case where a scanning trajectory is shifted.

An observation apparatus according to the technology of the disclosure comprises an imaging unit that images an observation target accommodated in a container, a movement unit that scans the observation target using an observation region of the imaging unit by relatively moving at least one of the container or the imaging unit with respect to the other in accordance with a predetermined scheduled trajectory, a measurement unit that acquires measured shape information of the container by measuring a shape of the container along a scanning trajectory of the observation target using the observation region, a storage unit that stores reference shape information acquired by measuring the shape of the container using the measurement unit along a reference trajectory which is a reference in a case where the shape of the container is measured in accordance with the scheduled trajectory, a calculation unit that calculates a shift of the scanning trajectory with respect to the reference trajectory based on the reference shape information and the measured shape information, and a control unit that corrects the scheduled trajectory based on the shift and scans the observation target using the observation region by controlling the movement unit based on the corrected scheduled trajectory.

The observation region is a region that can be observed by the imaging unit in a range of a field of view. The scheduled trajectory is a predetermined trajectory and represents a trajectory on which at least one of the container or the imaging unit is scheduled to be relatively moved with respect to the other. The scanning trajectory is a trajectory on which the observation region is actually relatively moved with respect to the container in a case where the observation target is scanned using the observation region by relatively moving at least one of the container or the imaging unit with respect to the other in accordance with the scheduled trajectory. The reference trajectory is a trajectory on which the observation region is relatively moved with respect to the container in a case where the observation target is scanned in the container as assumed using the observation region by relatively moving at least one of the container or the imaging unit with respect to the other in accordance with the scheduled trajectory. For example, the container as assumed is a container in a state where the container is appropriately placed at a position for measurement without inclination.

In the observation apparatus, an accommodation part that accommodates the observation target is formed in the container, and the measurement unit acquires the measured shape information by measuring a shape of a bottom surface of the accommodation part along the scanning trajectory.

In the observation apparatus, the calculation unit may calculate an inclination of the scanning trajectory with respect to the reference trajectory as the shift based on the reference shape information and the measured shape information.

In the observation apparatus, the control unit may determine a direction in which the scanning trajectory is inclined with respect to a proceeding direction of the reference trajectory based on an image in which the observation region is imaged by the imaging unit, and correct the scheduled trajectory based on the direction in which the scanning trajectory is inclined and the shift calculated by the calculation unit.

An observation method according to the technology of the disclosure comprises an imaging step of imaging an observation target accommodated in a container using an observation region, a measurement step of acquiring measured shape information of the container by measuring a shape of the container along a scanning trajectory of the observation target using the observation region in the imaging step in accordance with a predetermined scheduled trajectory, a storage step of storing reference shape information acquired by measuring the shape of the container in the measurement step along a reference trajectory which is a reference in a case where the shape of the container is measured in accordance with the scheduled trajectory, a calculation step of calculating a shift of the scanning trajectory with respect to the reference trajectory based on the reference shape information and the measured shape information, and a control step of correcting the scheduled trajectory based on the shift and performing imaging close to the reference trajectory in the imaging step based on the corrected scheduled trajectory.

An observation program according to the technology of the disclosure causes a computer to execute an imaging step of imaging an observation target accommodated in a container using an observation region, a measurement step of acquiring measured shape information of the container by measuring a shape of the container along a scanning trajectory of the observation target using the observation region in the imaging step in accordance with a predetermined scheduled trajectory, a storage step of storing reference shape information acquired by measuring the shape of the container in the measurement step along a reference trajectory which is a reference in a case where the shape of the container is measured in accordance with the scheduled trajectory, a calculation step of calculating a shift of the scanning trajectory with respect to the reference trajectory based on the reference shape information and the measured shape information, and a control step of correcting the scheduled trajectory based on the shift and performing imaging close to the reference trajectory in the imaging step based on the corrected scheduled trajectory.

Another observation apparatus according to the technology of the disclosure comprises a memory that stores an instruction to be executed by a computer, and a processor configured to execute the stored instruction. The processor executes an imaging step of imaging an observation target accommodated in a container using an observation region, a measurement step of acquiring measured shape information of the container by measuring a shape of the container along a scanning trajectory of the observation target using the observation region in the imaging step in accordance with a predetermined scheduled trajectory, a storage step of storing reference shape information acquired by measuring the shape of the container in the measurement step along a reference trajectory which is a reference in a case where the shape of the container is measured in accordance with the scheduled trajectory, a calculation step of calculating a shift of the scanning trajectory with respect to the reference trajectory based on the reference shape information and the measured shape information, and a control step of correcting the scheduled trajectory based on the shift and performing imaging close to the reference trajectory in the imaging step based on the corrected scheduled trajectory.

According to the technology of the disclosure, the shift of the scanning trajectory with respect to the reference trajectory is calculated from the reference shape information indicating the shape of the container acquired along the reference trajectory in accordance with the scheduled trajectory and the measured shape information obtained by actually measuring the container along the scanning trajectory, and the scheduled trajectory is corrected. Imaging is performed close to the reference trajectory controlling the movement unit and the imaging unit based on the corrected scheduled trajectory. Accordingly, even in a case where the scanning trajectory is shifted from the reference trajectory, the image can be appropriately captured using the observation region of the imaging unit by correcting the scheduled trajectory.

**BRIEF DESCRIPTION OF THE DRAWINGS**

Fig. 1 is a diagram illustrating a schematic configuration of an observation apparatus according to an embodiment of the technology of the disclosure.

Fig. 2 is a diagram illustrating one example of a placing stand.

Fig. 3 is a block diagram illustrating a configuration of a control unit according to the embodiment of the technology of the disclosure.

Fig. 4 is a diagram illustrating a scanning trajectory by a solid line M in a cultivation container.

Fig. 5 is a diagram illustrating a positional relationship among a first displacement sensor, a second displacement sensor, and the cultivation container in a case where an observation region is present at any position in the cultivation container.

Fig. 6 is a diagram illustrating a positional relationship among the first displacement sensor, the second displacement sensor, and the cultivation container in a case where the observation region is present at any position in the cultivation container.

Fig. 7 is a diagram illustrating an example of a two-dimensional shape of a container detected in an autofocus detection unit on a forward path.

Fig. 8 is a diagram illustrating an example of inclination of the scanning trajectory with respect to the cultivation container.

Fig. 9 is a flowchart illustrating a flow of observation method executed by the observation apparatus.

Fig. 10 is a diagram illustrating a shift of the scanning trajectory with respect to a reference trajectory.

Fig. 11 is a partial enlarged view of Fig. 10.

## DESCRIPTION OF THE PREFERRED EMBODIMENTS

Hereinafter, one example of an embodiment according to the technology of the disclosure will be described with reference to the drawings. The same or equivalent constituents and parts in each drawing will be designated by the same reference signs. Dimensional ratios in the drawings are exaggerated for convenience of description and may be different from the actual ratios.

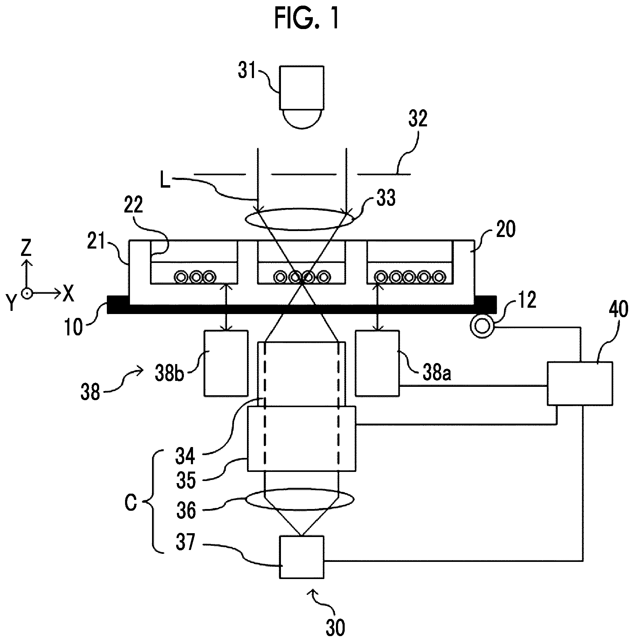

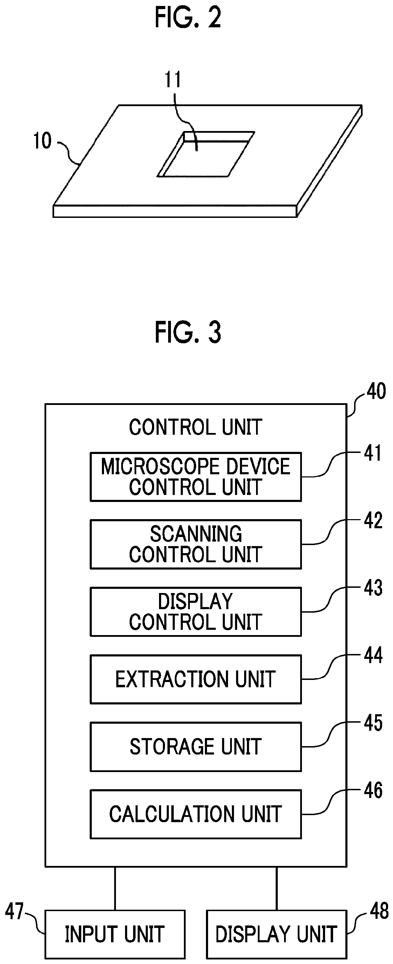

Fig. 1 is a diagram illustrating a schematic configuration of an observation apparatus according to the embodiment of the technology of the disclosure. Fig. 2 is a diagram illustrating one example of a placing stand.

The observation apparatus is an apparatus for observing an observation target accommodated in a cultivation container 20 placed on a placing stand 10 by a microscope device 30. The placing stand 10 and the microscope device 30 are controlled by a control unit 40. Each configuration will be described in order.

The placing stand 10 is a stage on which the cultivation container 20 can be placed. As illustrated in Fig. 2, a rectangular opening 11 is formed at the center of the placing stand 10. It is configured that the cultivation container 20 is installed on a member forming the opening 11, and light for observation by the microscope device 30 passes through the cultivation container 20.

A movement unit 12 is attached to the placing stand 10. The movement unit 12 can freely move the placing stand 10 in an X direction and a Y direction that are orthogonal to each other. The X direction and the Y direction are directions orthogonal to a Z direction and are directions orthogonal to each other in a horizontal plane. In the present embodiment, the X direction is set as a main scanning direction, and the Y direction is set as a sub-scanning direction. The movement unit 12 is configured with an actuator that includes a piezoelectric element or the like. Movement of the placing stand 10 in an X-Y plane is controlled by the control unit 40. By moving the placing stand 10 in the X-Y plane, the cultivation container 20 on the placing stand 10 moves with respect to the microscope device 30.

In the present embodiment, an example in which a position at which the observation target is observed by the microscope device 30 is changed by moving the placing stand 10 with respect to the microscope device 30 is illustrated. However, the example is not for limitation purposes. The microscope device 30 may be moved with respect to the placing stand 10, or both of the placing stand 10 and the microscope device 30 may be moved. Any aspect can be employed as long as at least one of the cultivation container 20 placed on the placing stand 10 or the microscope device 30 is relatively moved with respect to the other. In the present disclosure, for example, the "microscope device 30 is represented as relatively moving with respect to the cultivation container 20" even in a case where a position of the microscope device 30 is fixed and only the cultivation container 20 is moving. In addition, in the present disclosure, a trajectory accompanied by the relative movement is represented as a "scanning trajectory" even in a case where any of the microscope device 30 and the cultivation container 20 is actually moving.

Instead of placing the cultivation container 20 on the placing stand 10 and moving the cultivation container 20, the cultivation container 20 may be moved in the X-Y plane using a holding unit that holds at least a part of the cultivation container 20 by moving the holding unit.

In the cultivation container 20, a plurality of accommodation parts 22 are formed in a plate 21 having a flat plate shape. For example, a Petri dish, a dish, or a well plate can be used as the cultivation container 20. For example, the accommodation part 22 is a recessed portion having a circular shape in a plan view and is referred to as a well. The accommodation part 22 accommodates the observation target such as various cells immersed in a cultivation liquid. Cells accommodated in the accommodation part 22 include pluripotent stem cells such as an iPS cell and an ES cell, cells of a nerve, skin, cardiac muscle, and a liver that are differentiation-induced from a stem cell, cells of skin, a retina, cardiac muscle, a blood cell, a nerve, and an organ extracted from a human body, and the like.

The microscope device 30 captures a phase difference image of the observation target. In order to obtain a high magnification image, the microscope device 30 captures partial images of the observation target and the cultivation container 20 using an observation region smaller than each accommodation part 22 of the cultivation container 20. As described above, by moving the cultivation container 20 with respect to the microscope device 30, the microscope device 30 scans the cultivation container 20, and a series of partial images is obtained.

The microscope device 30 comprises a light source 31, a slit 32, a condenser lens 33, an objective lens 34, a focus adjustment mechanism 35, an image forming lens 36, an imaging unit 37, and a measurement unit 38.

The light source 31 emits white light. The slit 32 is formed by disposing a ring shaped slit through which the white light is transmitted in a light screen that blocks the white light emitted from the light source 31. Illumination light L having a ring shape is formed by causing the white light to pass through the slit. The condenser lens 33 condenses the illumination light L having the ring shape on the observation target.

The objective lens 34 is arranged to face the condenser lens 33 through the cultivation container 20. The objective lens 34 forms an image of the observation target in the cultivation container 20. The focus adjustment mechanism 35 includes a phase difference lens that can be moved in an optical axis direction (Z direction). By moving the phase difference lens in the optical axis direction, autofocus control is performed, and contrast of the phase difference image captured by the imaging unit 37 is adjusted. For example, the movement of the phase difference lens in the optical axis direction can be implemented by driving an actuator such as a piezoelectric element based on a signal from the control unit 40. However, the piezoelectric element is not for limitation purposes, and the phase difference lens can be driven using other known configurations as long as the phase difference lens can be moved in the Z direction. In addition, a magnification of the phase difference lens may be configured to be changeable. Specifically, a phase difference lens or the focus adjustment mechanism 35 having a different magnification may be configured to be replaceable. The replacement may be automatically performed or may be manually performed by a user.

The phase difference image that passes through the focus adjustment mechanism 35 is incident on the image forming lens 36, and the image forming lens 36 forms the phase difference image on the imaging unit 37.

The imaging unit 37 captures the phase difference image formed by the image forming lens 36. For example, the imaging unit 37 is an imaging element such as a charge-coupled device (CCD) image sensor or a complementary metal-oxide semiconductor (CMOS) image sensor. As the imaging element, an imaging element in which color filters of red, green, blue (RGB) are disposed may be used, or a monochrome imaging element may be used. The imaging unit 37 images a region observable in the range of the field of view as the observation region.

Hereinafter, the objective lens 34, the focus adjustment mechanism 35, the image forming lens 36, and the imaging unit 37 will be collectively referred to as an image forming optical system C.

The measurement unit 38 is fixedly attached with respect to the imaging unit 37 and detects a Z-directional position of the cultivation container 20 installed on the placing stand 10. Furthermore, the measurement unit 38 measures the shape of the cultivation container 20 by scanning the cultivation container 20 using the imaging unit 37 and the measurement unit 38 by relatively moving at least one of the cultivation container 20 or the imaging unit 37 with respect to the other and continuously detecting the Z-directional position of the cultivation container 20. In the present embodiment, the measurement unit 38 projects measurement light (laser light) toward the cultivation container 20 and detects reflection caused by a difference in refractive index between the cultivation container 20 and the cultivation liquid accommodated in the accommodation part 22. Thus, the measurement unit 38 measures a shape of a bottom surface of the accommodation part 22 of the cultivation container 20. The measurement unit 38 acquires measured shape information by relatively moving with respect to the observation region of the imaging unit 37 and the cultivation container 20 in accordance with a scheduled trajectory and measuring the shape of the cultivation container 20 along the scanning trajectory.

Specifically, the measurement unit 38 comprises a first displacement sensor 38a and a second displacement sensor 38b. The first displacement sensor 38a and the second displacement sensor 38b are arranged in the X direction illustrated in Fig. 1 with the image forming optical system C interposed therebetween. The first displacement sensor 38a and the second displacement sensor 38b in the present embodiment are laser displacement meters and detect a Z-directional position of a bottom surface of the cultivation container 20 by irradiating the cultivation container 20 with laser light and detecting reflected light. The bottom surface of the cultivation container 20 is a boundary surface between a bottom portion of the cultivation container 20 and the cell which is the observation target, that is, an observation target installation surface.

Z-directional positional information of the cultivation container 20 detected by the measurement unit 38 is output to the control unit 40. The control unit 40 performs the autofocus control by controlling the focus adjustment mechanism 35 based on the input positional information. The detection of the position of the cultivation container 20 by the first displacement sensor 38a and the second displacement sensor 38b, and the autofocus control will be described in detail later.

Next, a configuration of the control unit 40 controlling the microscope device 30 will be described. Fig. 3 is a block diagram illustrating a configuration of the control unit according to the embodiment of the technology of the disclosure.

The control unit 40 controls the entire microscope device 30 as described above and executes various processes. The control unit 40 includes a microscope device control unit 41, a scanning control unit 42, a display control unit 43, an extraction unit 44, a storage unit 45, a calculation unit 46, an input unit 47, and a display unit 48. The control unit 40 is configured with a computer that comprises a central processing unit (CPU), a semiconductor memory, and the like. In the control unit 40, an observation program according to one embodiment of the present invention is installed in the storage unit 45. The microscope device control unit 41, the scanning control unit 42, and the display control unit 43 illustrated in Fig. 3 function by causing the CPU to execute the observation program.

The microscope device control unit 41 controls the focus adjustment mechanism 35 based on the Z-directional positional information of the cultivation container 20 detected by the measurement unit 38 as described above. By driving the focus adjustment mechanism 35, the phase difference lens moves in the optical axis direction, and the autofocus control is performed.

In addition, the microscope device control unit 41 controls imaging performed by the imaging unit 37 in a case where the cultivation container 20 is scanned. Basically, a timing of imaging during scanning is stored in advance in the storage unit 45. The microscope device control unit 41 performs imaging based on the stored timing.

The scanning control unit 42 controls driving of the movement unit 12 and moves the placing stand 10 in the X direction and the Y direction.

The display control unit 43 generates one composite image by combining the series of partial images captured by the microscope device 30 and displays the composite image on the display unit 48.

The extraction unit 44 extracts the shape of the cultivation container 20 included in the image obtained by imaging performed by the imaging unit 37. Particularly, in the present embodiment, the extraction unit 44 extracts the shape of the edge of the accommodation part 22 of the cultivation container 20 included in the image.

The storage unit 45 stores the observation program that implements each function unit. In addition, the storage unit 45 stores container information related to the cultivation container 20. For example, the container information of the cultivation container 20 includes specifications (a position of the accommodation part 22, a size of the accommodation part 22, and the like) of the cultivation container and a model number, a maker, and the like of the cultivation container. Information of the number (6, 24, 96) of accommodation parts 22 of the currently used cultivation container 20, intervals of the accommodation parts 22, a diameter of the accommodation part 22, a thickness of the accommodation part 22, and the like is obtained from the container information. The storage unit 45 may store information indicating a solid shape of the cultivation container 20 obtained by measurement performed in advance by a shape measurement device such as a laser length measuring device. The information indicating the solid shape of the cultivation container 20 includes a shape of the bottom surface of the cultivation container 20, the position of the accommodation part 22 in the cultivation container 20, and the like.

Information indicating the scheduled trajectory of the microscope device 30 and the series of imaging positions (coordinates) on the scheduled trajectory is decided by the scanning control unit 42 based on the container information or the information indicating the solid shape of the cultivation container 20 and is stored in the storage unit 45. Furthermore, the storage unit 45 stores reference shape information indicating the shape of the cultivation container 20 when the cultivation container 20 is measured by the measurement unit 38 in a case where the cultivation container 20 is appropriately placed on the placing stand 10 as assumed and at least one of the cultivation container 20 or the imaging unit 37 is relatively moved with respect to the other along the scheduled trajectory. As described above, in a case where at least one of the cultivation container 20 placed as assumed or the imaging unit 37 is relatively moved with respect to the other along the scheduled trajectory, a trajectory on which the imaging unit 37 relatively moves with respect to the cultivation container 20 will be referred to as a reference trajectory.

The calculation unit 46 calculates a shift of the trajectory (scanning trajectory) on which the observation region of the microscope device 30 scans the cultivation container 20 with respect to the reference trajectory based on the reference shape information of the cultivation container 20 stored in the storage unit 45 and the measured shape information which is a measurement result obtained by measurement performed by the measurement unit 38. Details will be described later.

The input unit 47 comprises a mouse, a keyboard, and the like and receives various necessary data and various setting inputs from the user. For example, the input unit 47 of the present embodiment receives an input of data related to the reference shape information of the cultivation container 20 and the imaging positions.

The display unit 48 comprises, for example, a liquid crystal display and displays a composite phase difference image generated by the display control unit 43 as described above. The display unit 48 may be configured with a touch panel and double as the input unit 47.

Next, movement control of the placing stand 10 by the scanning control unit 42 and control of the microscope device 30 by the microscope device control unit 41 will be described in detail.

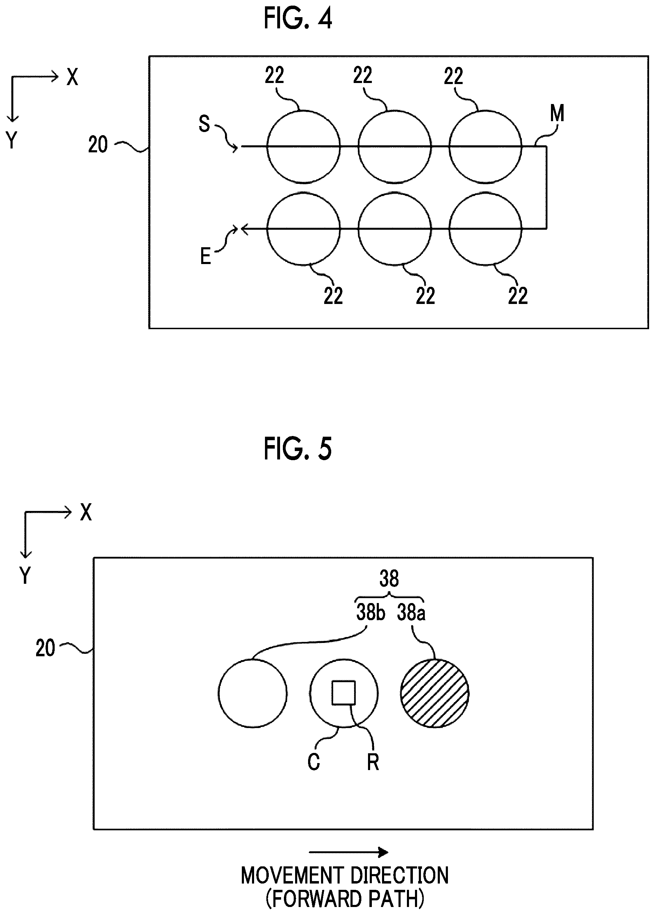

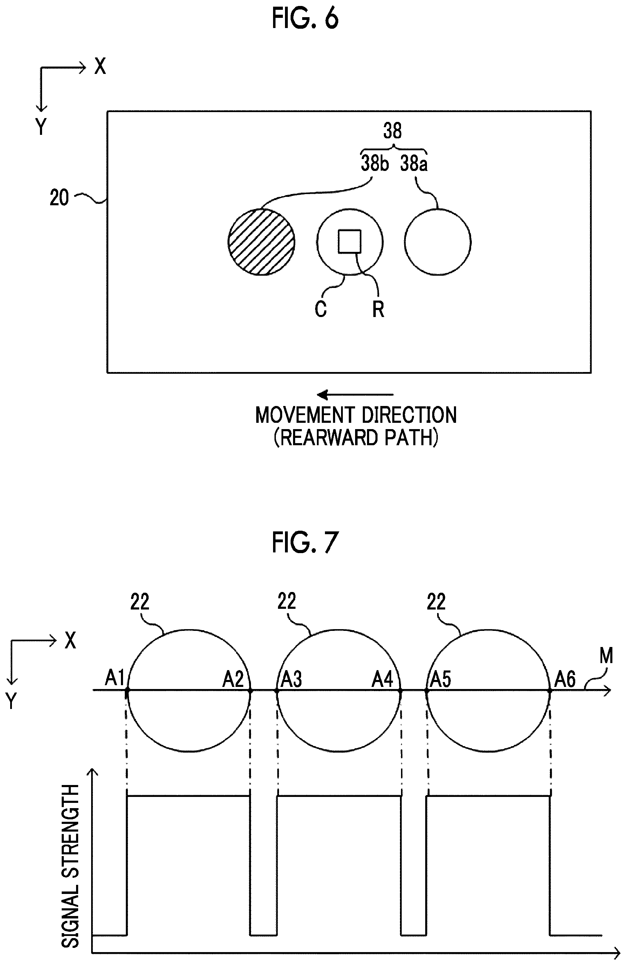

Fig. 4 is a diagram illustrating the scanning trajectory by a solid line M in the cultivation container. Fig. 5 and Fig. 6 are diagrams illustrating a positional relationship among the first displacement sensor, the second displacement sensor, and the cultivation container in a case where the observation region is present at any position in the cultivation container. Fig. 7 is a diagram illustrating an example of a two-dimensional shape of a container detected in an autofocus detection unit on a forward path.

In the present embodiment, the placing stand 10 is moved in the X direction and the Y direction under control of the scanning control unit 42, and the microscope device 30 scans the inside of the cultivation container 20 in two dimensions. During the scanning, partial images of the cultivation container 20 and the observation target are captured in each observation region of the microscope device 30. In the present embodiment, a well plate that includes six accommodation parts 22 is used as the cultivation container 20.

As illustrated in Fig. 4, the observation region of the microscope device 30 moves along the solid line M from a scanning start point S to a scanning end point E. That is, the observation region is scanned in a positive direction (a rightward direction in Fig. 4) of the X direction and then, moves in the Y direction (a downward direction in Fig. 4) and is scanned in the opposite negative direction (a leftward direction in Fig. 4). Next, the observation region moves in the Y direction again and is scanned in the positive direction again. By repeating reciprocation of the observation region in the X direction and movement of the observation region in the Y direction, the inside of the cultivation container 20 is scanned in two dimensions.

In the present embodiment, as illustrated in Fig. 5 and Fig. 6, the first displacement sensor 38a and the second displacement sensor 38b are arranged in the X direction with the image forming optical system C interposed therebetween. An observation region R of the image forming optical system C scans the inside of the cultivation container 20 in two dimensions as described above. At this point, the Z-directional position of the cultivation container 20 is detected at a position that is further in a movement direction of the observation region R than a position of the observation region R of the image forming optical system C with respect to the cultivation container 20. Specifically, in a case where the observation region R is moving in an arrow direction (a rightward direction in Fig. 5) illustrated in Fig. 5, the Z-directional position of the cultivation container 20 is detected by the first displacement sensor 38a that is further in the movement direction of the observation region R between the first displacement sensor 38a and the second displacement sensor 38b. In a case where the observation region R moves from the position illustrated in Fig. 5 to a position of the first displacement sensor 38a, the autofocus control is performed using the previously detected Z-directional positional information of the cultivation container 20, and the partial images are captured.

In a case where the observation region R is moving in an arrow direction (a leftward direction in Fig. 6) in Fig. 6, the Z-directional position of the cultivation container 20 is detected by the second displacement sensor 38b that is further in the movement direction of the observation region R between the first displacement sensor 38a and the second displacement sensor 38b. In a case where the observation region R moves from the position illustrated in Fig. 6 to a position of the second displacement sensor 38b, the autofocus control is performed using the previously detected Z-directional positional information of the cultivation container 20, and the phase difference images are captured.

The detection of the cultivation container 20 using the first displacement sensor 38a and the detection of the cultivation container 20 using the second displacement sensor 38b are switched depending on the movement direction of the observation region R. Accordingly, the Z-directional positional information of the cultivation container 20 at the position of the observation region R can be acquired at all times prior to capturing of the phase difference image of the observation region R.

Based on the Z-directional positional information of the cultivation container 20 detected beforehand as described above, the microscope device control unit 41 performs the autofocus control by controlling driving of the focus adjustment mechanism 35. Specifically, a relationship between the Z-directional positional information of the cultivation container 20 and a movement amount of the image forming optical system C in the optical axis direction is set in advance in the microscope device control unit 41. The microscope device control unit 41 obtains the movement amount of the image forming optical system C in the optical axis direction based on the input Z-directional positional information of the cultivation container 20 and outputs a control signal corresponding to the movement amount to the focus adjustment mechanism 35. The focus adjustment mechanism 35 is driven based on the input control signal. Accordingly, the phase difference lens is moved in the optical axis direction, and focus adjustment corresponding to the Z-directional position of the cultivation container 20 is performed.

The measurement performed by the measurement unit 38 is also used for other than the focus adjustment. The two-dimensional shape of the cultivation container 20 illustrated in a lower part of Fig. 7 is obtained by causing the measurement unit 38 to perform scanning along the scanning trajectory M and measure the Z-directional position of the cultivation container 20. In an upper part of Fig. 7, a part of the cultivation container 20 in a plan view is illustrated, and the scanning trajectory M in a case where the measurement unit 38 scans three linearly arranged accommodation parts 22. In the lower part of Fig. 7, the shape of the cultivation container 20 along the scanning trajectory M is illustrated by signal strength received by the measurement unit 38. As illustrated in Fig. 7, at a position at which the accommodation part 22 of the cultivation container 20 is present, the measurement light is reflected by the accommodation part 22. Thus, the signal strength is high. At a position at which the accommodation part 22 is not present, reflection of the measurement light is low, and the signal strength is low. That is, the shape of the cultivation container 20 measured as a waveform in which the signal strength is high at a part corresponding to the accommodation part 22 and the signal strength is low at the other part. The measurement result of the measurement unit 38 is sequentially stored in the storage unit 45.

As illustrated in Fig. 7, in a case where an arrangement direction of the accommodation parts 22 of the cultivation container 20 coincides with a direction of the scanning trajectory M, a waveform in which repetition of roughness is regular is measured. The arrangement direction of the accommodation parts 22 of the cultivation container 20 may not coincide with the direction of the scanning trajectory M. For example, a case where the cultivation container 20 is arranged at an inclination with respect to the placing stand 10 and the scanning trajectory M is inclined with respect to the arrangement direction of the accommodation parts 22 is present.

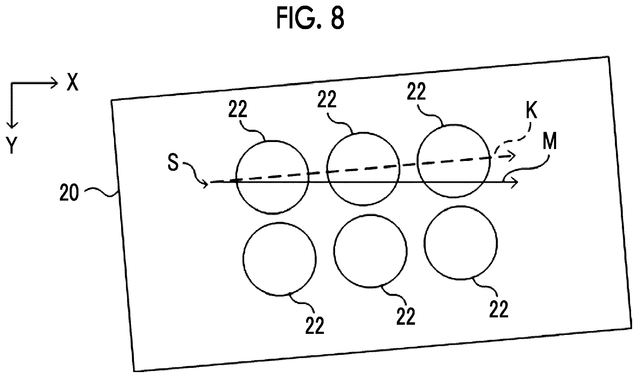

Fig. 8 is a diagram illustrating an example of inclination of the scanning trajectory with respect to the arrangement direction of the accommodation parts 22 of the cultivation container 20. In the case illustrated in Fig. 8, even in a case where imaging is performed using the image forming optical system C along the scanning trajectory, a scheduled part of the accommodation part 22 cannot be imaged, and the observation target also cannot be appropriately imaged. The observation apparatus according to the embodiment of the present disclosure performs the following control such that the scanning trajectory M is corrected using the shape of the cultivation container 20 measured by the measurement unit 38 even in a case where the scanning trajectory M is inclined with respect to the arrangement direction of the accommodation parts 22 of the cultivation container 20. An algorithm illustrated below is implemented by causing the CPU to execute the program stored in the storage unit 45.

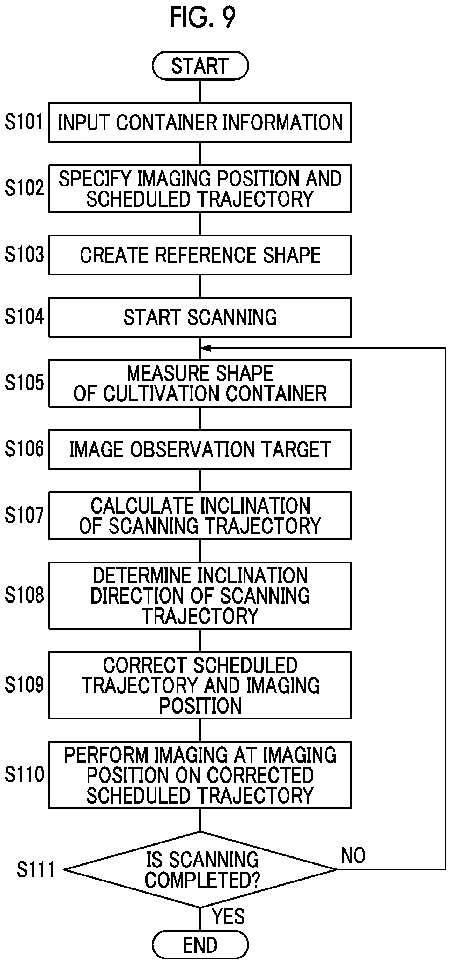

Fig. 9 is a flowchart illustrating a flow of observation method executed by the observation apparatus. Each step is particularly executed by the control unit 40.

First, the control unit 40 receives an input of the container information related to the cultivation container 20 in the input unit 47 (step S101). For example, the container information of the cultivation container 20 includes specifications (the size of the accommodation part 22, the number of accommodation parts 22, and the like) of the cultivation container and the model number, the maker, and the like of the cultivation container. Information of the number of accommodation parts 22 of the currently used cultivation container 20, the intervals of the accommodation parts 22, the diameter of the accommodation part 22, and the like is obtained from the container information. Even with the same specifications, arrangement positions and the like of the accommodation parts 22 may vary depending on the maker. Thus, information on the maker is also useful for specifying the shape of the cultivation container 20.

Next, the control unit 40 specifies the imaging positions and the scheduled trajectory from the container information obtained in step S101 (step S102). The imaging positions are specified as coordinate positions at which the observation target accommodated in the cultivation container 20 can be observed. For example, the control unit 40 specifies coordinates of the image forming optical system C at which the observation target in the accommodation part 22 can be observed as the imaging positions by assuming a state where the cultivation container 20 of which the shape is specified in step S101 is appropriately placed on the placing stand 10. In addition, the control unit 40 specifies a trajectory connecting the imaging positions as the scheduled trajectory representing a trajectory on which the imaging unit 37 is scheduled to be relatively moved with respect to the cultivation container 20. The control unit 40 may first decide the scheduled trajectory based on the container information and specify the coordinates of the imaging positions on the scheduled trajectory. The scheduled trajectory is preferably configured with straight lines as far as possible except for a time when the direction is changed.

A case where scanning is performed on the cultivation container 20 as assumed in accordance with the scheduled trajectory is scanning that is a reference along the arrangement of the accommodation parts 22. A trajectory of the reference with respect to the cultivation container 20 is the reference trajectory. For example, the cultivation container as assumed is a container in a state where the container is appropriately placed at a position for measurement without inclination. A trajectory on which the imaging unit 37 and the measurement unit 38 are actually relatively moved with respect to the cultivation container 20 along the scheduled trajectory will be referred to as the scanning trajectory below.

Even in a case where the cultivation container 20 is scanned in accordance with the scheduled trajectory, the actual scanning trajectory of the measurement unit 38 does not coincide with the reference trajectory with respect to the cultivation container 20 in a case where the cultivation container 20 is placed at an inclination on the placing stand 10. In a case where the cultivation container 20 is appropriately placed on the placing stand 10 without inclination, the actual scanning trajectory coincides with the reference trajectory in a case where the cultivation container 20 is scanned in accordance with the scheduled trajectory. Thus, the reference trajectory may be a reference with respect to the scanning trajectory which is the actual scanning trajectory. The reference trajectory includes a part that is parallel to the arrangement direction of the accommodation parts 22 of the cultivation container 20.

Next, as the reference shape information, the control unit 40 creates shape information of the cultivation container 20 that may be obtained in a case where it is assumed that the shape of the cultivation container 20 is measured by the measurement unit 38 along the reference trajectory decided in step S102 by appropriately placing the cultivation container 20 based on the container information obtained in step S101 (step S103). In this step, the reference shape information may be obtained by actually measuring the correctly placed cultivation container 20 using the laser length measurement device or the measurement unit 38, or the reference shape information may be calculated based on the container information and the reference trajectory without actual measurement. Step S101 to step S103 are pre-processing of observation performed by the observation apparatus. Accordingly, step S101 to step S103 may be executed in advance separately from step S104 and subsequent steps.

The control unit 40 starts scanning the cultivation container 20 by moving the placing stand 10 in accordance with the scheduled trajectory predetermined in step S102 using the scanning control unit 42 and starting imaging performed by the microscope device 30 (step S104). The imaging performed by the microscope device 30 accompanies measurement of the shape of the cultivation container 20 performed by the measurement unit 38 as described above.

The control unit 40 measures the shape of the cultivation container 20 using the measurement unit 38 (step S105) and images the observation target using the image forming optical system C at the imaging positions (step S106).

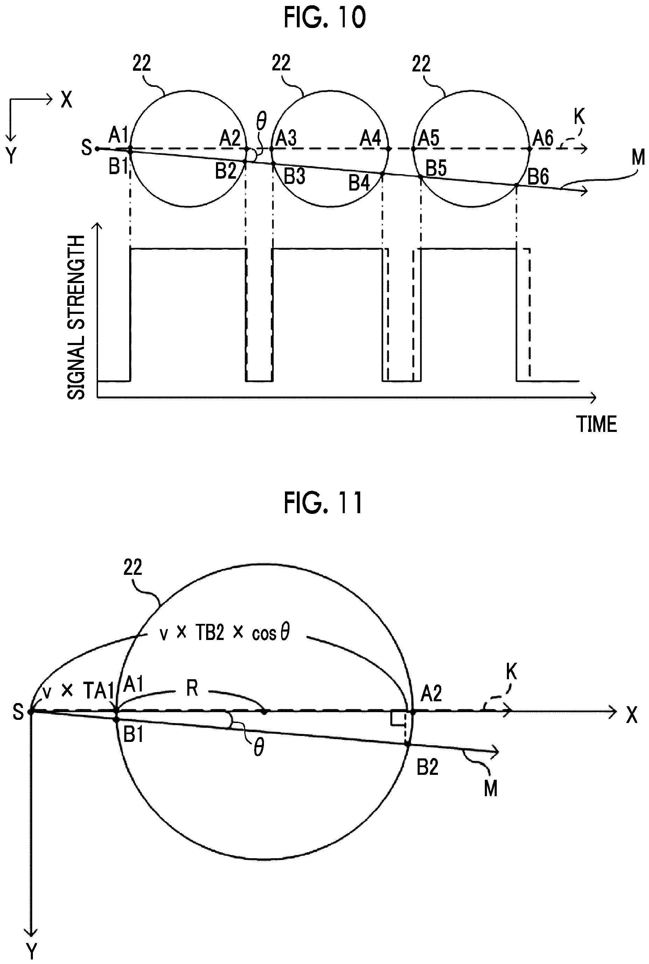

The control unit 40 calculates an inclination of the scanning trajectory with respect to the reference trajectory as a shift of the actual scanning trajectory with respect to the reference trajectory (step S107). A specific method of calculating the inclination of the scanning trajectory will be described later with reference to Fig. 10.

The control unit 40 determines a direction in which the scanning trajectory is inclined with respect to a proceeding direction of the reference trajectory (step S108). For example, in the example illustrated in Fig. 8, the scanning trajectory m is inclined to the right side in a view from a proceeding direction of a reference trajectory K illustrated by a dotted line. In order to determine an inclination side, for example, the control unit 40 checks the image in which the observation region is imaged by the imaging unit 37. As illustrated in Fig. 8, in a case where the scanning trajectory M is inclined in a rightward direction (right side) of a scanning direction, the image captured by the imaging unit 37 is shifted in a positive Y direction (lower side in Fig. 8). Such a shift can be determined in a case where the shape of the edge of the accommodation part 22 in the image is checked. Accordingly, an inclination direction of the scanning trajectory M can be determined by extracting the shape of the edge of the accommodation part 22 in the image captured by the imaging unit 37 using the extraction unit 44.

The control unit 40 corrects the scheduled trajectory and the imaging positions based on the inclination and the inclination direction of the scanning trajectory M obtained in step S107 and step S108 (step S109).

The control unit 40 relatively moves the imaging unit 37 to the corrected imaging positions by controlling the movement unit 12 based on the corrected scheduled trajectory and imaging positions and performs imaging using the imaging unit 37 (step S110).

Next, the control unit 40 determines whether or not the scanning is completed to the end (step S111) and returns to the process of step S105 in a case where the scanning is not completed yet (step S111: NO). In a case where the scanning is completed to the end (step S111: YES), the control unit 40 finishes an observation process.

Next, calculation of the inclination of the scanning trajectory will be specifically described with reference to Fig. 10 and Fig. 11.

Fig. 10 is a diagram illustrating a shift of the scanning trajectory with respect to the reference trajectory. Fig. 11 is a partial enlarged view of Fig. 10.

In an upper part of Fig. 10, a part of the cultivation container 20 in a plan view is illustrated. The scanning trajectory M in a case where the measurement unit 38 actually performs scanning is illustrated by a solid line arrow, and the reference trajectory K is illustrated by a dotted line arrow. In a lower part of Fig. 10, the shape information (measured shape information) of the cultivation container 20 along the scanning trajectory M is illustrated by a solid line, and the shape information (reference shape information) of the cultivation container 20 along the reference trajectory K is illustrated by a dotted line.

As illustrated in the upper part of Fig. 10, the reference trajectory K intersects with the edges of the accommodation parts 22 at positions A1 to A6 in the arrangement direction of the accommodation parts 22. In a case where the scanning trajectory M is inclined from the reference trajectory K, the scanning trajectory M is also inclined from the arrangement direction of the accommodation parts 22 and intersects with the edges of the accommodation parts 22 at positions B1 to B6.

Consequently, as illustrated in the lower part of Fig. 10, a part in which the accommodation part 22 is present and a part in which the accommodation part 22 is not present regularly appear in the reference shape information of the cultivation container 20 measured by the measurement unit 38 along the reference trajectory K. That is, the reference shape information is a waveform in which a time in which the signal strength is high evenly appears. The part in which the accommodation part 22 is present and the part in which the accommodation part 22 is not present do not regularly appear in the measured shape information of the cultivation container 20 actually measured along the scanning trajectory M. In the measured shape information along the scanning trajectory M, a waveform in which a range in which the signal strength is high is decreased from the range in the shape information of the cultivation container along the reference trajectory K as the scanning proceeds is obtained.

For example, it is assumed that a time from the scanning start point S to A1 on the reference trajectory K is denoted by TA1, a time from the scanning start point S to B2 on the scanning trajectory M is denoted by TB2, and a relative movement speed of the placing stand 10 with respect to the imaging unit 37 is denoted by V. In addition, as illustrated in Fig. 11, it is assumed that a radius of the accommodation part 22 is denoted by R and an inclination angle θ of the scanning trajectory M with respect to the reference trajectory K is denoted by θ. An xy coordinate system in which the scanning start point S is an origin is considered.

In this case, the circular edge of the accommodation part 22 is represented by

$$\{x - (V \times TA1 + R)\}^2 + y^2 = R \ldots (1).$$

A straight line of the scanning trajectory M is represented by

$$y = x \times \tan \theta \ldots (2).$$

Substituting y of Expression (1) with x × tan θ of Expression (2) results in

$$\{x - (V \times TA1 + R)\}^2 + (x \times \tan \theta)^2 = R \ldots (3).$$

An x coordinate of the point B2 is obtained as

$$x = V \times TB2 \times \cos \theta \ldots (4).$$

In a case where x of Expression (3) is substituted with V × TB2 × cos θ of Expression (4), an expression in which θ is the only variable is obtained, and a value of θ can be calculated.

In a case where the inclination θ is obtained and the inclination side of θ is found in step S108, the positions B3, B4, B5, and B6 can be calculated without actually performing scanning to the positions B3, B4, B5, and B6 of the scanning trajectory M. Accordingly, the control unit 40 can calculate correction amounts Δx and Δy for correcting the position B3, the position B4, the position B5, and the position B6 to the position A3, the position A4, the position A5, and the position A6, respectively, and can correct the imaging positions based on the calculated correction amounts. The control unit 40 can also correct the scheduled trajectory in accordance with correction of the imaging positions. The control unit 40 preferably corrects sub subsequent imaging positions in accordance with the calculated correction amounts. However, in a case where the shift is large and correcting the imaging positions to completely remove the shift needs the movement unit 12 to be stopped or unreasonably driven or causes an unreasonable imaging timing of the imaging unit 37, correction may not be necessarily performed in accordance with the calculated correction amounts. In this case, the control unit 40 corrects the scheduled trajectory to approach the reference trajectory K as far as possible without causing unreasonable driving of the movement unit 12 and an unreasonable imaging timing of the imaging unit 37.

The method of calculating the inclination θ is one example, and θ may be calculated using any method. For example, while θ is calculated by actually performing measurement to the position B2 in the calculation method, the inclination θ may be calculated after measurement is performed to more positions such as the position B3 and the position B4.

Calculation of the actual shift of the scanning trajectory M with respect to the reference trajectory K may be performed only once immediately after imaging of one cultivation container 20 is started, or may be performed a plurality of times during imaging.

As described above, according to the embodiment of the present disclosure, the observation apparatus calculates the actual shift of the scanning trajectory M with respect to the reference trajectory K from the reference shape information indicating the shape of the cultivation container 20 along the reference trajectory K and the measured shape information obtained by measurement performed by the measurement unit 38. The observation apparatus controls the imaging positions and the movement direction set by the movement unit 12 such that the scanning trajectory M approaches the reference trajectory K. Accordingly, even in a case where the cultivation container 20 is not installed in a correct posture on the placing stand 10 and the scanning trajectory M is shifted from the reference trajectory K, the observation apparatus can capture the image close to an appropriate imaging position.

The measurement unit 38 measures the shape of the cultivation container 20 as a waveform showing the part in which the accommodation part 22 is present and the part in which the accommodation part 22 is not present. Accordingly, an error of the scanning trajectory can be easily calculated using a tendency of the waveform.

The control unit 40 determines the direction in which the scanning trajectory is inclined with respect to the proceeding direction of the reference trajectory K based on the image in which the observation region is imaged by the imaging unit 37. Accordingly, as illustrated in Fig. 10, even in a case where the reference trajectory K exactly passes through the diameter of the accommodation part 22, which side (in the example in Fig. 10, an upper side or a lower side) the obtained inclination $\theta$ is present with respect to the reference trajectory K can be determined. In a case where the reference trajectory K does not pass through the diameter of the accommodation part 22, the inclination direction of the inclination $\theta$ can be determined by only the waveform in Fig. 10 without using the image captured by the imaging unit 37.

In the embodiment, a case where the cultivation container 20 is not appropriately placed on the placing stand 10 has been described as a case where the scanning trajectory M is shifted from the reference trajectory K. However, the technology of the present disclosure can also be applied to a case where the scanning trajectory M is shifted from the reference trajectory K due to any other causes. For example, even in a case where the accommodation parts 22 are irregularly arranged due to a manufacturing error or the like in the cultivation container 20, the scheduled trajectory can be corrected in accordance with the arrangement direction of the accommodation parts 22, and scanning can be performed by the imaging unit 37 in the arrangement direction.

The observation process that is executed by causing the CPU to read software (program) in embodiment may be executed by various processors other than the CPU. In this case, the processors are illustrated by a programmable logic device (PLD) such as a field-programmable gate array (FPGA) of which a circuit configuration can be changed after manufacturing, a dedicated electric circuit such as an application specific integrated circuit (ASIC) that is a processor having a circuit configuration dedicatedly designed to execute a specific process, and the like. In addition, the observation process may be executed by one of the various processors or may be executed by a combination of two or more processors of the same type or different types (for example, a plurality of FPGAs and a combination of a CPU and an FPGA). In addition, a hardware structure of the various processors is more specifically an electric circuit in which circuit elements such as semiconductor elements are combined.

In embodiment, an aspect in which the program of the observation process is stored (installed) in advance in the storage unit 45 is described. However, the aspect is not for limitation purposes. The program may be provided in a form of a recording on a recording medium such as a compact disk read only memory (CD-ROM), a digital versatile disk read only memory (DVD-ROM), and a Universal Serial Bus (USB) memory. In addition, the program may be downloaded from an external device through a network.

Explanation of References

| 10: | placing stand |
|---|---|
| 11: | opening |
| 12: | movement unit |
| 20: | cultivation container |
| 21: | plate |
| 22: | accommodation part |
| 30: | microscope device |
| 31: | light source |
| 32: | slit |
| 33: | condenser lens |
| 34: | objective lens |
| 35: | focus adjustment mechanism |
| 36: | image forming lens |
| 37: | imaging unit |
| 38: | measurement unit |
| 38a: | first displacement sensor |
| 38b: | second displacement sensor |
| 40: | control unit |
| 41: | microscope device control unit |
| 42: | scanning control unit |
| 43: | display control unit |
| 44: | extraction unit |
| 45: | storage unit |
| 46: | calculation unit |
| 47: | input unit |

| 48: | display unit |
| A1 to A6, B1 to B6: | position |
| C: | image forming optical system |
| E: | scanning end point |
| K: | reference trajectory |
| L: | illumination light |
| M: | scanning trajectory |
| R: | observation region |
| S: | scanning start point |

**Claims**

1. An observation apparatus comprising:

an imaging unit that images an observation target accommodated in a container;
a movement unit that scans the observation target using an observation region of the imaging unit by relatively moving at least one of the container or the imaging unit with respect to the other in accordance with a predetermined scheduled trajectory;
a measurement unit that acquires measured shape information of the container by measuring a shape of the container along a scanning trajectory of the observation target using the observation region;
a storage unit that stores reference shape information acquired by measuring the shape of the container using the measurement unit along a reference trajectory which is a reference in a case where the shape of the container is measured in accordance with the scheduled trajectory;
a calculation unit that calculates a shift of the scanning trajectory with respect to the reference trajectory based on the reference shape information and the measured shape information; and
a control unit that corrects the scheduled trajectory based on the shift and scans the observation target using the observation region by controlling the movement unit based on the corrected scheduled trajectory.

2. The observation apparatus according to claim 1,

wherein an accommodation part that accommodates the observation target is formed in the container, and
the measurement unit acquires the measured shape information by measuring a shape of a bottom surface of the accommodation part along the scanning trajectory.

3. The observation apparatus according to claim 1 or 2,
wherein the calculation unit calculates an inclination of the scanning trajectory with respect to the reference trajectory as the shift based on the reference shape information and the measured shape information.

4. The observation apparatus according to claim 3,
wherein the control unit determines a direction in which the scanning trajectory is inclined with respect to a proceeding direction of the reference trajectory based on an image in which the observation region is imaged by the imaging unit, and corrects the scheduled trajectory based on the direction in which the scanning trajectory is inclined and the shift calculated by the calculation unit.

5. An observation method comprising:

an imaging step of imaging an observation target accommodated in a container using an observation region;
a measurement step of acquiring measured shape information of the container by measuring a shape of the container along a scanning trajectory of the observation target using the observation region in the imaging step in accordance with a predetermined scheduled trajectory;
a storage step of storing reference shape information acquired by measuring the shape of the container in the measurement step along a reference trajectory which is a reference in a case where the shape of the container is measured in accordance with the scheduled trajectory;
a calculation step of calculating a shift of the scanning trajectory with respect to the reference trajectory based on the reference shape information and the measured shape information; and
a control step of correcting the scheduled trajectory based on the shift and performing imaging close to the reference trajectory in the imaging step based on the corrected scheduled trajectory.

6. An observation program causing a computer to execute:

an imaging step of imaging an observation target accommodated in a container using an observation region;
a measurement step of acquiring measured shape information of the container by measuring a shape of the container along a scanning trajectory of the observation target using the observation region in the imaging step in accordance with a predetermined scheduled trajectory;
a storage step of storing reference shape information acquired by measuring the shape of the container in the measurement step along a reference trajectory which is a reference in a case where the shape of the container is measured in accordance with the scheduled trajectory;
a calculation step of calculating a shift of the scanning trajectory with respect to the reference trajectory based on the reference shape information and the measured shape information; and
a control step of correcting the scheduled trajectory based on the shift and performing imaging close to the reference trajectory in the imaging step based on the corrected scheduled trajectory.

# FIG. 1

# FIG. 2

11

10

# FIG. 3

40

CONTROL UNIT

| MICROSCOPE DEVICE CONTROL UNIT | 41 |

| SCANNING CONTROL UNIT | 42 |

| DISPLAY CONTROL UNIT | 43 |

| EXTRACTION UNIT | 44 |

| STORAGE UNIT | 45 |

| CALCULATION UNIT | 46 |

47 INPUT UNIT     DISPLAY UNIT 48

# FIG. 4

# FIG. 5

MOVEMENT DIRECTION
(FORWARD PATH)

# FIG. 6

X

Y

20

38

38b 38a

C    R

MOVEMENT DIRECTION
(REARWARD PATH)

# FIG. 7

22    22    22

X

M

Y

A1    A2  A3    A4  A5    A6

SIGNAL STRENGTH

# FIG. 8

# FIG. 9

```
                    ( START )
                        │
 S101 │ INPUT CONTAINER INFORMATION │
                        │
 S102 │ SPECIFY IMAGING POSITION AND
        SCHEDULED TRAJECTORY │
                        │
 S103 │ CREATE REFERENCE SHAPE │
                        │
 S104 │ START SCANNING │
                        │ ◄──────────────┐
 S105 │ MEASURE SHAPE                    │
        OF CULTIVATION CONTAINER │       │
                        │                │
 S106 │ IMAGE OBSERVATION TARGET │       │
                        │                │
 S107 │ CALCULATE INCLINATION           │
        OF SCANNING TRAJECTORY │        │
                        │                │
 S108 │ DETERMINE INCLINATION           │
        DIRECTION OF SCANNING            │
        TRAJECTORY │                     │
                        │                │
 S109 │ CORRECT SCHEDULED               │
        TRAJECTORY AND IMAGING           │
        POSITION │                       │
                        │                │
 S110 │ PERFORM IMAGING AT IMAGING      │
        POSITION ON CORRECTED            │
        SCHEDULED TRAJECTORY │           │
                        │                │
 S111 ◄   IS SCANNING          NO        │
            COMPLETED? ───────────────────┘
                        │ YES
                    ( END )
```

# FIG. 10

# FIG. 11

## INTERNATIONAL SEARCH REPORT

| International application No. |
|---|
| PCT/JP2018/041976 |

A. CLASSIFICATION OF SUBJECT MATTER
Int.Cl. G02B21/00(2006.01)i, C12M1/34(2006.01)i, G02B21/06(2006.01)i,
G02B21/26(2006.01)i

According to International Patent Classification (IPC) or to both national classification and IPC

B. FIELDS SEARCHED

Minimum documentation searched (classification system followed by classification symbols)
Int.Cl. G02B21/00, C12M1/34, G02B21/06, G02B21/26

Documentation searched other than minimum documentation to the extent that such documents are included in the fields searched
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2017-161385 A (SCREEN HOLDINGS CO., LTD.) 14 September 2017, paragraphs [0069], [0091], [0092], fig. 30 & WO 2017/154253 A1 & TW 201732366 A | 1-6 |
| A | JP 2011-48013 A (NIKON CORP.) 10 March 2011, paragraphs [0124]-[0143], fig. 8-14 (Family: none) | 1-6 |

☐ Further documents are listed in the continuation of Box C.        ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 04 February 2019 (04.02.2019) | 19 February 2019 (19.02.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**EP 3 726 273 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016149998 A
- JP 2016099592 A
- WO 2008146474 A